Europäisches Patentamt

(19) European Patent Office

Office européen des brevets

(11) Numéro de publication: **0 223 285**
**B1**

(12) **FASCICULE DE BREVET EUROPEEN**

(45) Date de publication du fascicule du brevet:
06.09.89

(21) Numéro de dépôt: 86201910.6

(22) Date de dépôt: 03.11.86

(51) Int. Cl.⁴: **A61B 6/00**

(54) Statif d'exploration isocentrique.

(30) Priorité: 05.11.85 FR 8516375

(43) Date de publication de la demande:
27.05.87 Bulletin 87/22

(45) Mention de la délivrance du brevet:
06.09.89 Bulletin 89/36

(84) Etats contractants désignés:
DE FR GB NL

(56) Documents cités:
EP-A- 0 049 562

(73) Titulaire: PHILIPS SYSTEMES MEDICAUX, 177, rue de
Bezons, F-78420 Carrières sur Seine(FR)

(84) Etats contractants désignés: FR

(73) Titulaire: N.V. Philips' Gloeilampenfabrieken,
Groenewoudseweg 1, NL-5621 BA Eindhoven(NL)

(84) Etats contractants désignés: DE GB NL

(72) Inventeur: Loulday, André Emile, Société Civile
S.P.I.D. 209, rue de l'Université, F-75007 Paris(FR)

(74) Mandataire: Landousy, Christian et al, Société Civile
S.P.I.D. 209, Rue de l'Université, F-75007 Paris(FR)

ACTORUM AG

## Description

L'invention concerne un statif d'exploration isocentrique destiné aux examens radiologiques d'un patient comprenant.
- un arceau en forme d'arc de cercle déplaçable circulairement, dans un plan qui le contient, autour d'un axe de rotation passant par le centre de l'arc de cercle,
- un support d'arceau monté à rotation sur un axe parallèle au plan de l'arceau et muni d'un moyen de déplacement circulaire de l'arceau,
- une source de rayonnement X et un détecteur de rayonnement, supportés tous deux par l'arceau, placés de part et d'autre du support, et dont l'axe source-détecteur parallèle au plan de l'arceau coupe l'axe de rotation du support d'arceau en un point dit isocentre.

Un tel statif d'exploration est connu du brevet français n° 2 544 191. Le brevet français 2 544 191 décrit un statif pour exploration isocentrique, assurant à un arceau ouvert des conditions de déplacement qui permettent des incidences latérales ainsi que la reconstitution de l'axe de travail intial en remplaçant sur cet axe la source de rayonnement et le détecteur l'un par l'autre. Ces possibilités sont obtenue par un arceau supporté par un support mobile en forme d'arc de cercle, lui-même supporté par un socle et déplaçable circulairement par rapport à ce socle.

Les statifs d'exploration isocentrique sont en général réalisés en pièces fondues ou soudées puis ensuite usinées. Ces pièces sont de masses importantes en alliage d'aluminium ou en acier afin d'assurer une rigidité mécanique aux statifs dont l'arceau supporte le poids, en porte à faux, de la source de rayonnement X et du détecteur de rayonnement.

Le câble d'alimentation de la source et du détecteur est, soit suspendu à une potence soit incorporé dans l'anneau puis suspendu. De tels statifs comportent plusieurs inconvénients:
- la fabrication de ces statifs entrainent nécessairement un investissement important en outillage dans le cas d'une fabrication moulée ou soudée ainsi qu'un usinage coûteux des parties mécaniques fonctionnelles,
- le câble d'alimentation incorporé dans l'arceau entrainent un affaiblissement de la rigidité mécanique de l'arceau.
- le câble suspendu à une potence est d'un grand encombrement.

L'invention a pour but de réaliser un statif d'exploration isocentrique de conception simple, économique, sans l'utilisation d'accessoires et conçu sans outillage de fabrication complexe.

Conformément à l'invention le statif d'exploration isocentrique est caractérisé en ce que l'arceau est composé d'éléments semblables juxtaposés et liés mécaniquement les uns aux autres pour former une structure rigide ayant sensiblement la forme globale d'un arceau sur laquelle s'appuient et sont liés mécaniquement au moins une enveloppe extérieure et au moins une enveloppe intérieure formant avec la structure une portion de cylindre, la structure autorisant la fixation du câble d'alimentation de la source et du détecteur de rayons X et son assujettissement au support d'arceau.

La forme de l'arceau est prédéterminée par un ensemble d'éléments liés entre eux, par exemple par vis et écrou ou rivets, ces éléments réalisant entre eux une préforme. Sur cette préforme sont fixés les deux enveloppes et le câble d'alimentation de la source et du détecteur de rayonnement X, le passage du câble ne nuisant pas à la rigidité mécanique de l'arceau.

Dans une forme préférentielle de l'invention, les éléments semblables sont des tronçons de tubes cylindriques dont les axes longitudinaux sont parallèles à l'axe de rota tion de l'arceau. Ces éléments peuvent aussi former par exemple des polygones réguliers ou toutes autres formes, éléments qui juxtaposés et liés entre eux préformeront un arceau.

Particulièrement, les enveloppes intérieure et extérieure sont chacune formée d'une tôle roulée rectangulaire faisant saillie de part et d'autre des éléments semblables juxtaposés. La préforme réalisée avec les éléments liés entre eux permet de porter er de former intérieurement et extérieurement une bande de tôle qui épouse l'enveloppe géométrique de la préforme.

De plus, les bords longitudinaux des enveloppes intérieure et extérieure en saillie sont des bandes de roulement et de guidage de l'arceau dans le support d'arceau. Les bandes de tôle roulées de largeur supérieure à la longueur des tubes cylindriques, constituant par exemple la préforme, dépassent latéralement, de part et d'autre desdits tubes, pour former un arceau de section en H, les ailes du H constituants les bandes de roulement qui guidant l'arceau dans son plan de déplacement autour de son axe de rotation.

Dans une autre forme particulière de l'invention, le câble d'alimentation de la source et du détecteur de rayons X fixés sur les éléments juxtaposés parcourt l'arceau pour sortir dans une zone séparant ledit arceau en deux arcs égaux et entrer dans le support d'arceau en formant une boucle. Le passage du câble dans la partie médiane de l'arceau permet à celui-ci de se déplacer en rotation extrême dans son plan en réduisant au maximum la longueur du câble formant la boucle.

Préférentiellement le support d'arceau est monté à rotation sur un chemin de roulement circulaire formant une couronne. Le câble d'alimentation qui pénêtre dans le support d'arceau sort dans l'axe de rotation du support, par un passage vide centré dans l'axe de la couronne pour accéder aux source fixes d'alimentation.

La description qui suit et les dessins illustrent un exemple de réalisation de l'invention.

La figure 1 est une vue d'ensemble en perspective d'un statif d'exploration isocentrique.
La figure 2 est une vue suivant la flèche A de la figure 1.
La figure 3 est une coupe en section droite suivant la flèche B de l'arceau.

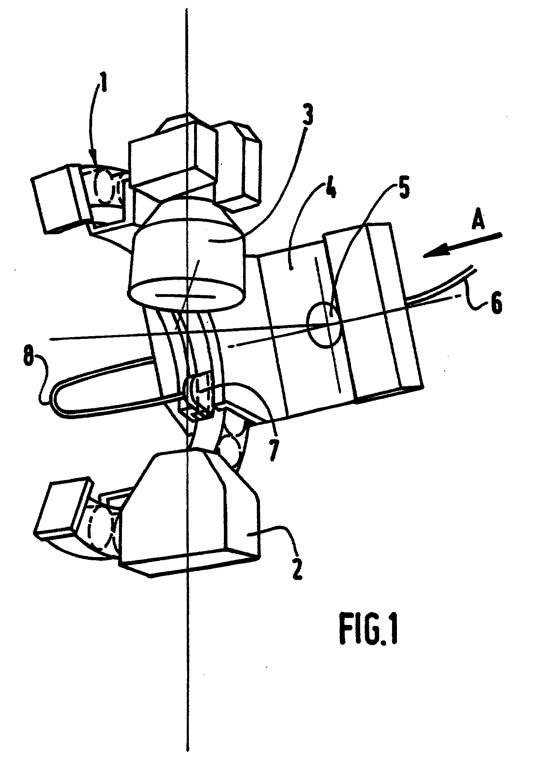

La figure 1 est une vue d'ensemble en perspective d'un statif d'exploration isocentrique comprenant un arceau 1 sur lequel sont montés, diamétralement opposés une source 2 de rayonnement X et un détecteur 3 de rayonnement X comme par exemple un tube intensificateur d'image. L'arceau 1 est porté par un support 4 d'arceau monté à rotation autour d'un axe 5 horizontal, axe contenu dans le plan défini par un axe source 2-détecteur 3 parallèle au plan de rotation de l'arceau et qui se déplace comme lui circulairement. L'intersection de l'axe 5 et de l'axe source-détecteur est un point appelé isocentre.

Un câble 6 d'alimentation électrique de la source 2 et du détecteur 3 est fixé par une bride 7 et forme une boucle 8 avant de pénétrer dans le support d'arceau 4.

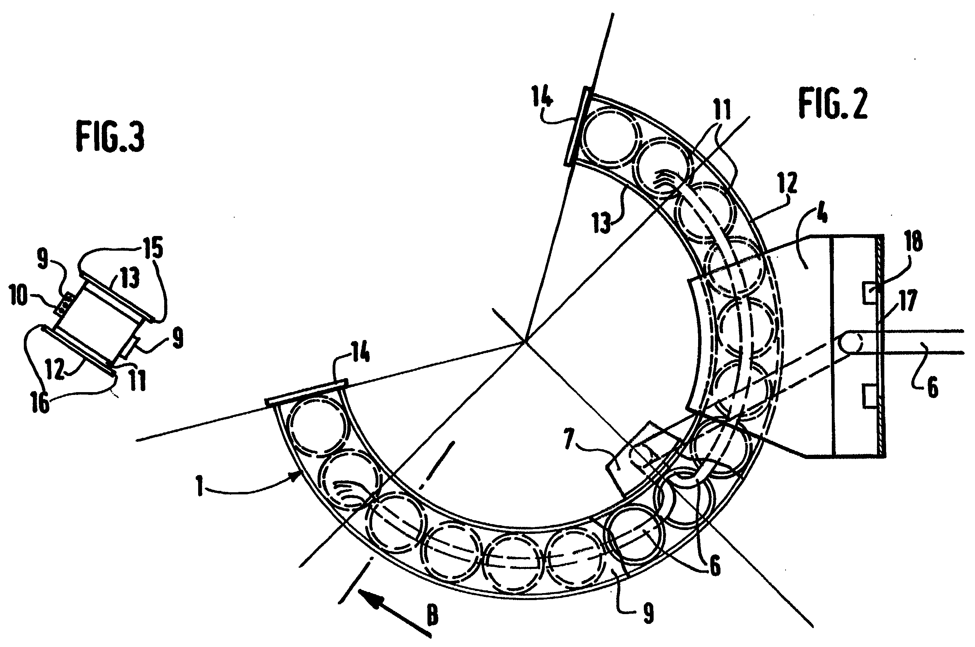

La figure 2 est une vue suivant la flèche A (figure 1) du statif auquel il a été retiré la source 2, le détecteur 3 et partiellement un cache câble 9 afin de laisser apparaître le chemin parcouru de câble 6 d'alimentation de la source X et du détecteur.

L'arceau est composé de tronçons de cylindre 11 vus sur la figure en bout et formant alors chacun deux cercles concentriques. Les cylindres sont liés entre eux par des pièces mécaniques non représentées sur le dessin pouvant être par exemple des vis et écrou ou des rivets, les cylindres ayant été prépercés avec une grande précision afin qu'en les liant les uns aux autres le perçage et l'assemblage réalisent et imposent à chacun des cylindres 11 une position définissant entre eux, avec précision, une structure ayant la forme globale d'un arceau.

La structure alors préformée avec l'ensemble des cylindres 11 porte au moins une enveloppe extérieure 12 et au moins une enveloppe intérieure 13 prépercées qui s'appuient sur des génératrices des cylindres 11 pour former un arceau représentant parfaitement un cylindre creux de dimensions prédéterminées, calibrées. L'arceau ouvert forme un secteur cylindrique de 240° afin que la source 2 et le détecteur 3 puissent être positionnés de manière diamétralement opposée. Deux flasques 14 ferment les deux sections en bout du secteur cylindrique.

La forme de l'arceau ainsi obtenue n'exige qu'un perçage de précision sans fonderie ni soudure, les chemins de roulement sont obtenus directement avec les enveloppes intérieure 13 et extérieure 12.

L'arceau 1 est monté sur un support 4 d'arceau dans lequel il coulisse sur des galets pout son déplacement circulaire, les galets; dont l'axe de rotation est parallèle à l'axe de rotation de l'arceau; pinçant les deux bords latéraux 15, 16, en saillie, de chacune des deux enveloppes 12 et 13.

Le câble 6 est posé et fixé en appui sur la structure formée des cylindres 11, puis recouvert d'un cache 9 fermant les sections de l'arceau cylindrique. Le câble arrive sur une bride 7 montée sur l'enveloppe intérieure 13 et placée de façon à séparer l'arceau en deux arcs sensiblement égaux. Seul un trou de passage est réalisé sur l'enveloppe intérieure, le passage du câble ainsi réalisé n'affaiblit pas la rigidité mécanique de l'arceau.

En plaçant la bride 7 dans une zone médiane de l'arceau, le câble sortant de l'arceau pour pénétrer dans le support 4 d'arceau forme une boucle de longueur minimale, la longueur de la boucle étant liée à la distance séparant la bride 7 du support 4 lorsque le déplacement de l'arceau est extrême.

Le passage du câble 6 dans le support 4 d'arceau lié à la construction de l'arceau 1 et à la position de la bride 7 impose que le câble puisse ressortir vers les sources d'énergie électrique (haute tension, secteur). Dans l'exemple de réalisation conforme à l'invention le câble 6, amené dans le support 4 d'arceau, passe dans un trou 17 aménagé dans le milieu d'un chemin de roulement 18 en forme de couronne. Le chemin de roulement 18 est par exemple un axe de rotation circulaire à galet définissant l'axe 5 de rotation du support 4 d'arceau.

La figure 3 est une vue en coupe de l'arceau suivant la flèche B montrant un cylindre 11, en coupe longitudinale, les enveloppes intérieure 13 et extérieure 12 qui s'appuient sur une génératrice du cylindre et qui font saillie aux deux extrémités du cylindre pour former bande de roulement, et aussi deux flasques 9 cachant le câble 6 d'alimentation de la source de rayonnement X et du détecteur.

## Revendications

1. Statif d'exploration isocentrique destiné aux examens radiologiques d'un patient comprenant
- un arceau en forme d'arc de cercle déplaçable circulairement, dans un plan qui le contient, autour d'un axe de rotation passant par le centre de l'arc de cercle,
- un support d'arceau monté à rotation sur un axe parallèle au plan de l'arceau et muni d'un moyen de déplacement circulaire de l'arceau,
- une source de rayonnement X et un détecteur de rayonnement, supportés tous deux par l'arceau, placés de part et d'autre du support, et dont l'axe source-détecteur parallèle au plan de l'arceau coupe l'axe de rotation du support d'arceau en un point dit isocentre,
caractérisé en ce que l'arceau est composé d'éléments semblables juxtaposés et liés mécaniquement les uns aux autres pour former une structure rigide ayant sensiblement la forme globale d'un arceau sur laquelles s'appuient et sont liés mécaniquement au moins une enveloppe extérieure et au moins une enveloppe intérieure formant avec la structure une portion de cylindre, la structure autorisant la fixation d'un câble d'alimentation de la source et du détecteur de rayons X et son assujettissement au support d'arceau.

2. Statif selon la revendication 1, caractérisé en ce que les éléments semblables sont des tronçons de tubes cylindriques dont les axes longitudinaux sont parallèles à l'axe de rotation de l'arceau.

3. Statif selon la revendication 1, caractérisé en ce que les enveloppes intérieure et extérieure sont chacune formée d'une tôle roulée rectangulaire faisant saillie de part et d'autre des éléments semblables juxtaposés.

4. Statif selon les revendications 1 ou 3, caractérisé en ce que les bords longitudinaux des enveloppes intérieure et extérieure en saillie sont des bandes de roulement et de guidage de l'arceau dans le support d'arceau.

5. Statif selon la revendication 1, caractérisé en ce que le câble d'alimentation de la source et du détecteur de rayons X fixés sur les éléments juxtaposés parcourt l'arceau pour sortir dans une zone séparant ledit arceau en deux arcs égaux et entrer dans le support d'arceau en formant une boucle.

6. Statif selon la revendication 1, caractérisé en ce que le support d'arceau est monté à rotation sur un chemin de roulement circulaire formant une couronne.

## Patentansprüche

1. Stativ für isozentrische Untersuchungen zum Durchleuchten eines Patienten mit Röntgenstrahlen, mit
– einem kleinen, mit einer Kreisbewegung in einer Ebene verschiebbaren Kreisbogen um eine durch die Mitte des Kreisbogens gehende Drehungsachse,
– einem zum Drehen auf einer zur Bogenebene parallel verlaufenden Achse montierten Bogenträger mit einer in einem Kreis bewegbaren Bogenverlagerungseinrichtung,
– einer Röntgenstrahlungsquelle und einem Strahlungsdetektor, die beide vom Bogen getragen werden und an beiden Seiten des Trägers angeordnet sind, wobei die parallel zur Bogenebene verlaufende Achse Quelle/Detektor die Trägerdrehungsachse in einem sog. isozentrischen Punkt schneidet, dadurch gekennzeichnet, daß der Bogen aus gleichen, nebeneinandergestellten und mechanisch miteinander verbundenen Elementen zur Bildung einer starren und im wesentlichen bogenförmigen Struktur besteht, auf der sich wenigstens eine Außenhülle und wenigstens eine Innenhülle zur Bildung eines zylinderförmigen Teils zusammen mit der Struktur abstützen und mechanisch miteinander verbunden sind, wobei die Struktur die Befestigung eines Speisekabels der Quelle und des Röntgendetektors und seine starre Befestigung auf dem Bogenträger ermöglicht.

2. Stativ nach Anspruch 1, dadurch gekennzeichnet, daß die gleichen Elemente zylindrische Rohrschüsse sind, deren Längsachsen parallel zur Drehungsachse des Bogens verlaufen.

3. Stativ nach Anspruch 1, dadurch gekennzeichnet, daß die Innen- und Außenhüllen aus je einem rechteckig gestanzen, an beiden Seiten der gleichen nebeneinandergestellten Elementen auskragenden Blech besteht.

4. Stativ nach Anspruch 1 oder 3, dadurch gekennzeichnet, daß die Längsränder der auskragenden Innen- und Außenhüllen Lauf- und Leitflächen des Bogens im Bogenträger sind.

5. Stativ nach Anspruch 1, dadurch gekennzeichnet, daß das Speisekabel der Röntgenquelle und des Röntgendetektors, die auf den nebeneinandergestellten Elementen befestigt sind, den Bogen durchläuft, wonach es in einem Gebiet austritt, das den Bogen in zwei gleiche Halbbögen trennt und unter Schleifenbildung in den Bogen eintritt.

6. Stativ nach Anspruch 1, dadurch gekennzeichnet, daß der Bogenträger zur Drehung auf einer kreisförmigen Rollbahn montiert ist, die einen Kranz bildet.

## Claims

1. An isocentric examination stand for radiological examinations of a patient comprising
– a cradle in the form of an arc of a circle circularly displaceable in a plane containing it about an axis of rotation passing through the centre of the arc of a circle,
– a cradle support mounted for rotation on an axis parallel to the plane of the cradle and provided with means for circularly displacing the cradle,
– an X-ray radiation source and a radiation detecor both supported by the cradle disposed on either side of the support, while the axis of source and detector parallel to the plane of the cradle intersects the axis of rotation of the cradle support and the so-called isocentre point, characterized in that the cradle is composed of similar juxtaposed elements mechanically connected to each other so as to form a rigid structure of guides substantially having the global form of a cradle, on which bear and are mechanically connected at least one external envelope and at least one internal envelope forming with the structure a cylinder portion, the structure permitting of fixing the supply cable of the source and the X-ray detector and of securing it to the cradle support.

2. A stand as claimed in Claim 1, characterized in that the similar elements are portions of a cylindrical tube whose longitudinal axes are parallel to the axis of rotation of the cradle.

3. A stand as claimed in Claim 1, characterized in that the internal and external envelopes are each constituted by a rolled rectangular plate projecting on either side from the similar juxtaposed elements.

4. A stand as claimed in Claims 1 or 3, characterized in that the projecting longitudinal edges of the internal and external envelopes are rolling strips for guiding the cradle in the cradle support.

5. A stand as claimed in Claim 1, characterized in that the supply cable of the source and of the X-ray detector fixed on the juxtaposed elements traverses the cradle and then leaves it at a zone dividing the said cradle into two equal arcs and enters the cradle support whilst forming a ring.

6. A stand as claimed in Claim 1, characterized in that the cradle support is mounted for rotation on a circular rolling track forming a crown.

FIG.1

FIG.3

FIG.2

EP 0 223 285 B1